**Europäisches Patentamt**

**European Patent Office**

**Office européen des brevets**

(11) Publication number: **0 286 359**
**A2**

# EUROPEAN PATENT APPLICATION

(21) Application number: **88303026.4**

(22) Date of filing: **05.04.88**

(51) Int. Cl.⁴: **A61B 8/06 , H01L 41/08 , G01F 1/66**

(30) Priority: **10.04.87 US 36796**

(43) Date of publication of application:
**12.10.88 Bulletin 88/41**

(84) Designated Contracting States:
**CH DE FR LI**

(71) Applicant: **CARDIOMETRICS, INC.**
**645 Clyde Avenue**
**Mountain View California 94043(US)**

(72) Inventor: **Haase, Wayne C.**
**2764 Doverton Square**
**Mountain View California 94940(US)**
Inventor: **Segal, Jerome**
**2884 Cowper Street**
**Palo Alto California 94306(US)**
Inventor: **Corl, Paul D.**
**264 Ventura Avenue**
**Palo Alto California 94306(US)**

(74) Representative: **Cross, Rupert Edward Blount**
**et al**
**BOULT, WADE & TENNANT 27 Furnival Street**
**London EC4A 1PQ(GB)**

(54) **Apparatus, system and method for measuring volumetric flow of blood in a vessel.**

(57) System for measuring the volumetric flow of blood in a vessel of a patient comprising a transducer positioned in a vessel in a patient for supplying ultrasonic energy. The transducer produces a substantially uniform beam which encompasses the vessel. The transducer receives ultrasonic energy back scattered from the red blood cells. A first moment detector is provided. A signal is supplied from the transducer to the first moment detector. A calculated correctional factor is also provided to the output of the first moment detector to provide an electrical output representing volume flow.

FIG. — 1

# APPARATUS, SYSTEM AND METHOD FOR MEASURING VOLUMETRIC FLOW OF BLOOD IN A VESSEL

This invention relates generally to an apparatus, system and method for measuring volumetric flow of blood in a vessel and more particularly, to such an apparatus, system and method using ultrasonics.

Diagnostic catheters have heretofore been provided for measuring intravascular pressure and blood flow using thermal dilution, dye dilution and oxygen consumption methods. More recently, intravascular catheters have been developed which measure instantaneous flow velocity utilizing ultrasonic Doppler transducers to measure the "Doppler shift" created by movement of red blood cells, acting as targets, within the blood vessel or organ to which a measurement is being made. Doppler systems of that character have been described extensively in the literature, as for example, in Cole et al "Pulsed Doppler Coronary Artery Catheter", Circulation, Vol. 56, No. I, July 1977 and Sibley et al, "Subselective Measurement of Coronary Blood Flow Velocity Using A Steerable Doppler Catheter" Journal of American College of Cardiology (Vol. 8, No. 6 December 1986 1332-40). Typically the Doppler catheters utilized in such systems are useful only for the measurement of flow velocity within a small sample volume contained within the blood vessel of interest. Volumetric flow cannot be accurately determined utilizing such Doppler systems. Additional information such as vessel dimensions, flow profile and incidence angle between the ultrasound beam and flow must be known in order to accurately determine volumetric flow. Cutaneous Doppler systems have been developed which allow volumetric flow measurements to be performed and which are independent of Doppler angle, vessel size and velocity profile. These cutaneous Doppler systems utilize a uniform insonification method as taught by Hottinger and Meindl and described in "Blood Flow Measurement Using Attenuation-Compensated Volume Flowmeter" Ultrasonic Imaging, Vol. I, No. I, 1979 and U. S. Patent Nos. 4,007,236, 3,888,238, 4,431,936 and 4,519,260. Volumetric flow measurements made with such a method require the use of two coplanar ultrasonic beams which are precisely positioned within the vessel of interest. Typically, a large uniform beam in the near field of a relatively large, cutaneous acoustic transducer is used to insonify the entire dimension in the blood vessel of interest. A second coplanar reference beam is contained entirely within the blood vessel of interest is used to generate a scale factor for conversion of flow related parameters into a quantitative measurement of true flow. Problems have been encountered with such a method because of the difficulty of placing the two required ultrasonic beams in the desired precise locations with respect to the vessel. It is difficult to assure that the large uniform beam encompasses the entire blood vessel and that the reference beam is totally contained within the blood vessel. In addition it has been found that neighboring vessels, if contained within the measurement sample volume, will cause inaccuracies in flow measurement. Furthermore, additional errors may result because of different tissue attenuation characteristics encountered in the two ultrasonic paths. There is therefore a need for a new and improved apparatus, system and method for measuring volumetric flow of blood in a vessel which overcomes these difficulties.

In general, it is an object of the present invention to provide an apparatus, system and method for measuring volumetric flow of blood in a vessel.

Another object of the invention is to provide an apparatus, system and method of the above character in which uniform insonification is provided in the vessel in the far field of an acoustic transducer, such uniform beam completely covering the entire lumen of the vessel of interest.

Another object of the invention is to provide an apparatus, system and method of the above character which avoids interference from neighboring blood vessels.

Another object of the invention is to provide an apparatus, system and method of the above character which places a single transducer entirely within a blood vessel of interest in order to provide a single uniform beam which insonifies the entire vessel lumen and provides a signal for measurement of volumetric flow.

Another object of the invention is to provide an apparatus, system and method of the above character which utilizes a single transducer which is placed entirely within the blood vessel of interest and supplies a signal to a first moment detector via pulsed Doppler circuitry.

Another object of the invention is to provide an apparatus, system and method in which the first moment detector weights the various Doppler shift frequency components in accordance with the number of red blood cells (acoustic scatters) traveling at velocities normal to the plane of the sample volume of the ultrasonic beam in the blood vessel.

Another object of the invention is to provide an apparatus, system and method of the above character in which a calculated correctional factor is applied to the first moment signal obtained from the transducer via the pulsed Doppler circuitry to obtain a measurement which is representative of the volume of blood flow within the vessel.

Another object of the invention is to provide an apparatus, system and method of the above character in which intravascular blood flow measurements are made using pulsed Doppler circuitry, with little or no interference from other blood vessels beyond the boundries of the walls of the vessel of interest.

Another object of the invention is to provide an apparatus, system and method of the above character in which extremely small transducers are provided at the distal extremities of the flexible elongate element disposed within the vessel of interest.

Another object of the invention is to provide an apparatus in which the flexible elongate element is a guide wire.

Another object of the invention is to provide an apparatus, system and method in which intravascular Doppler volumetric diametric flow measurements are independent of angle and orientation of the transducer.

Another object of the invention is to provide an apparatus, system and method of the above character which is particularly suitable for use in angioplasty.

Additional objects and features of the present invention will appear from the following description in which the preferred embodiments are set forth in detail in conjunction with the accompanying drawings.

Figure I is a schematic illustration of a system and apparatus for measuring volumetric flow of blood in a vessel incorporating the present invention.

Figure 2 is an enlarged distal extremity of the flexible elongate element in the form of a balloon dilatation catheter shown in Figure I.

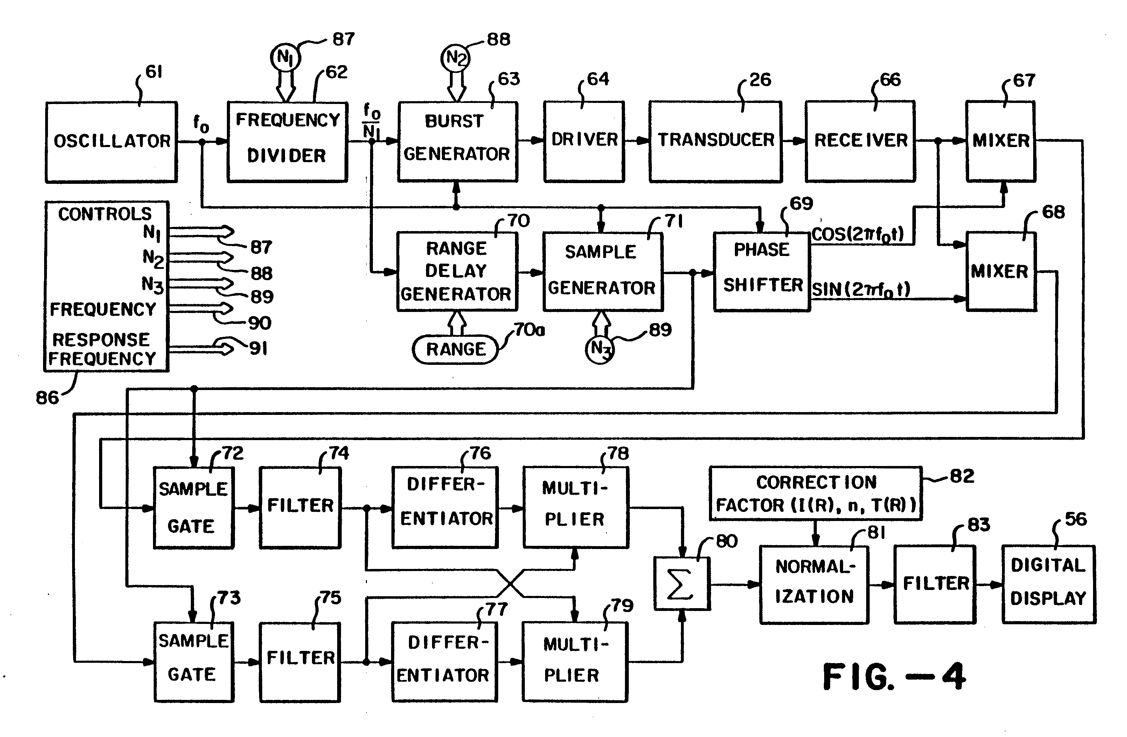

Figure 3 is an end elevational view of the balloon dilatation catheter shown in Figure 2 looking along the line 3-3 of Figure 2.

Figure 4 is a block diagram of the electronic circuitry utilized in the system and apparatus shown in Figure I.

Figure 5 is a timing diagram for the circuitry shown in Figure 4.

Figure 6 is a side elevational view of another embodiment of a flexible elongate element in the form of a guide wire.

Figure 7 is an enlarged cross sectional view of the distal extremity of the guide wire shown in Figure 6.

Figure 8 is a view taken along the lines 8-8 of Figure 7.

Figure 9 is a cross sectional view taken along the line 9-9 of Figure 7.

The system and method for measuring volumetric blood a vessel of a patient is comprised of a transducer which is disposed within the vessel. Means is provided for supplying radio frequency energy to the transducer to cause the transducer to create a beam of ultrasonic energy which encompasses the vessel. Ultrasonic energy reflected by the red blood cells in the vessel is received by the transducer and the Doppler shift frequency is converted to an electrical signal which is supplied to a first moment detector via pulsed Doppler circuitry. A calculated correctional factor is applied to the output from the first moment detector to provide an estimate of volume flow of blood through the vessel.

More in particular, the system II for measuring volumetric flow of blood in a vessel consists of a flexible elongate transducer carrying device I2 which is connected to a flow meter I3. The flexible elongate transducer carrying device I2 as shown in Figure I is in the form of a balloon dilatation catheter. The catheter can be constructed in the manner disclosed in patent No. 4,323,071. Thus it is provided with an inner flexible tubular member I6 having a lumen I7 extending therethrough. It also consists of an outer flexible tubular member I8 which is disposed coaxially on the inner flexible tubular member I6 and forms an annular flow passage or lumen I9. A balloon 2I is carried by the distal extremity of the outer member I8 and as shown can be formed integral therewith. The lumen I9 opens into the interior 22 of the balloon and is used for inflating and deflating the balloon 2I. The inner member I6 and the outer member I8 can be formed of suitable plastic such as polyethylene. It should be appreciated that if desired, rather than the co-axial construction hereinbefore described for the catheter, a single plastic flexible elongate tubular member can be provided having first and second lumens formed therein in which one serves as a guide wire lumen and the other serves as a balloon inflation lumen. In addition, it should be appreciated that if desired, a separate balloon can be provided rather than an integral balloon in which the separate balloon is bonded to the outer flexible tubular member I8.

A transducer 26 is carried by the distal extremity 27 of the inner flexible tubular member I6. The transducer 26 can be constructed of an appropriate piezoelectric material such as lead-titanate-zirconate ceramic. For reasons hereinafter described, the transducer is in the form of a toroid which has a central opening 28 that is in registration with the lumen I7. The transducer 26 is secured to the distal extremity 27 of the inner member I6 by a suitable adhesive such as a tungsten-oxide loaded epoxy backing material 29. The distal extremities 27 and 3I can be sealed with respect to each other in a suitable manner such as by heat shrinking the distal extremity 3I of the outer tubular member I8 onto the distal extremity 27 of the inner tubular member I6. Alternatively if desired, a suitable adhesive can be provided and wicked between the distal extremities 27 and 3l so that a liquid-tight seal is formed between the distal extremities of the members l6 and l8.

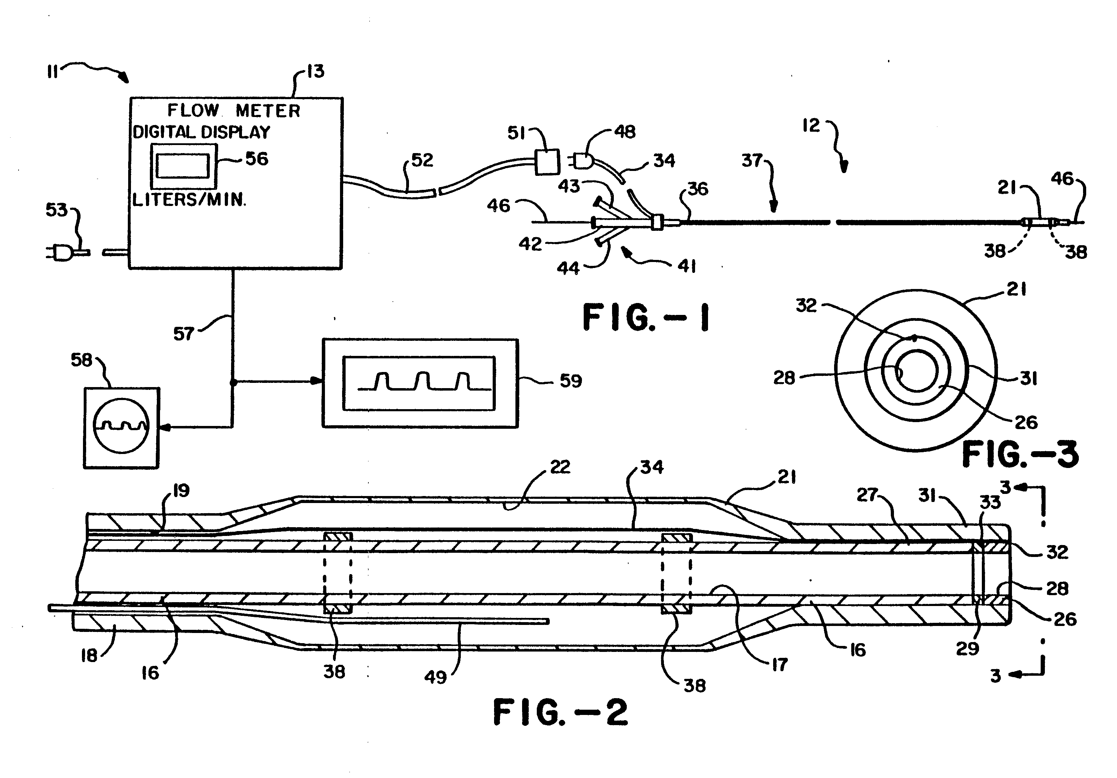

As can be seen from Figure 2, the transducer 26 is also supported by the outer distal extremity 3l of the outer flexible tubular member l8. Front and rear electrical connections 32 and 33 are provided on the transducer 26 and are connected to a two conductor wire 34 which extends rearwardly away from the distal extremity. The two conductor wire 34 is disposed between the outer tubular member l8 and the inner tubular member l6 and extends rearwardly through the balloon 2l and through the lumen l9 to the proximal extremity 36 of the catheter l2.

A pair of radiopaque markers 38 which are spaced apart within the balloon are carried by the inner tubular member l6 to facilitate visualizing of the balloon during use of the same. The markers 38 can be formed of a suitable material such as gold bands, or alternatively, as coils of platinum wire.

The inner tubular member l6 and the outer tubular member l8 as well as the toroidal transducer 26 can have suitable dimensions. For example, the lumen l7 in the inner tubular member can have a suitable dimension such as .02l inches as can the opening 28 in the toroidal transducer 26. The tubular member l6 can have an outer diameter of .03l inches whereas the outer tubular member l8 can have an outside diameter of .048 inches.

A three-arm adapter 4l of a conventional type is mounted on the proximal extremity 36 of a shaft 37 which is formed by the inner tubular member l6 and the outer tubular member l8. The tubular members l6 and l8 can have a suitable length as, for example, approximately l75 centimeters.

The three-arm adapter 4l is provided with a central arm 42 and side arms 43 and 44. The central arm 42 is in communication with the lumen l7 and the inner tubular member l6 is adapted to receive a guide wire 46 of a conventional construction which is utilized for facilitating placement of the catheter as hereinafter described. The side arm 43 can be utilized as an inflation and deflation port and is in communication with the annular flow passage l9 that is in communication with the interior 22 of the balloon 2l. The two conductor wire 34 can be brought out from the lumen l9 just distal of the three-arm adapter 4l as shown in Figure l and is connected to a male connector 48. The side arm 44 is also in communication with the lumen l9 and is adapted to receive a vent tube 49 of a conventional type which extends into the interior 22 of the balloon and is utilized for venting air from the balloon as the balloon is inflated.

The male connector 48 is adapted to be connected to a female connector l5 provided on cable 52 which is connected to the flow meter l3. The flow meter l3 is provided with a power cord which is adapted to be connected to a suitable source of 60 cycle ll0 volts AC. The flow meter l3 is provided with a digital display 56 which gives it readout in liters per minute. The flow meter l3 is also provided with an analog output which can be utilized in conjunction with an oscilloscope 58 and/or a strip chart recorder 59.

The flowmeter l3 includes the electronics which is shown in block diagram in Figure 4. As shown therein, the electronics consists of a plurality of blocks 6l through 83 which serve functions as hereinafter described. The apparatus also includes a control panel or computer 86 which provides control outputs 87 through 9l which are used in a manner hereinafter described.

Operation and use of the system and apparatus in performing the method of the present invention may now be briefly described as follows. Let it be assumed that the system and apparatus is to be utilized in conjunction with a balloon angioplasty procedure. In such a procedure, the balloon 2l is first inflated outside the body with a radiographic contrast liquid through the side arm 43. Air within the balloon is forced out through the balloon vent tube 49. After the balloon 2l has been completely inflated so there are no air bubbles in the same, the balloon is deflated. The guide wire 46 is inserted through the central arm 42 until it extends beyond the distal extremity of the catheter l2. The catheter l2 with the guide wire 46 therein can then be advanced into the blood vessel of the patient in a conventional manner so that the balloon is in the vicinity of the stenosis to be dilated. It then may be desirable to ascertain the volume blood flow through the stenosis prior to dilation. To accomplish this, the male connector 48 is connected into the female connector 5l and the flowmeter l3 is placed in operation.

In the making of volumetric flow measurements with flowmeter l3, utilizing a single beam, it is necessary that there be provided a relatively uniform beam which extends over the entire cross sectional area of the vessel being examined which can range in size form l millimeter to 35 millimeters. Assuming that the vessel in which the catheter l2 is positioned has a diameter of approximately 2.5 millimeters, a crystal or transducer 26 is utilized having a frequency of 6.7 MHz and an outer diameter of approximately 0.75 millimeters. A beam is produced with a divergence angle of approximately 20°. This divergence angle can be calculated approximately as follows:

Divergence angle = $\frac{\lambda}{D}$

where $\lambda = \frac{c}{f}$

where c = the velocity of sound which is

approximately 1.54 millimeters per microsecond

where D is the diameter of the transducer crystal in millimeters, and where f is the frequency of the crystal in megahertz

Using these formulas, the calculations can be made as follows:

$$\lambda = \frac{1 \cdot 54}{6 \cdot 7} \approx .25 \text{ mm.}$$

divergence angle $= \frac{\cdot 25}{\cdot 75} = \frac{1}{3}$ of a radian $\approx 20°$

Thus it can be seen with a transducer of that size and that frequency, a beam is produced which has a divergence angle greater than at least 15° as for example, from approximately 5° to 120°. At a divergence angle of approximately 20° with a range gate depth ranging from 1 to 40 millimeters and preferably at approximately 10 millimeters which is in the far field, flowmeter 13 in conjunction with transducer 26 will produce a relatively uniform beam over the cross-sectional area of a vessel having a diameter of approximately 2.5 millimeters. An ultrasonic beam of this dimension would thus encompass the entire lumen of an average coronary artery.

The oscillator 61 generates the desired frequency $f_0$ which with the present transducer is 6.7 MHz. This signal from the oscillator 61 is supplied to the frequency divider 62 which generates a signal at a frequency $f_0/N1$ where N1 represents the division provided by the frequency divider 62. N1 is determined by the output 87 from the computer 86. The outputs from the oscillator 61 and from the frequency divider 62 are both supplied to a burst generator 63 to provide a signal output which is comprised of N2 consecutive cycles at frequency $f_0$ and repeating every N1 cycles of the frequency $f_0$. The number N2 is supplied from the computer output 88.

The output from the burst generator 63 is supplied to the driver 64 which amplifies the signal and has an appropriate output impedance to drive the transducer 26 hereinbefore described. The transducer 26 converts the electrical drive signal from the driver 64 into an acoustic wave which travels through the blood within the vessel of interest. The output of the frequency divider 62 thus determines the repetition rate of the system, that is the rate at which the burst of acoustic-wave packets travel from the transducer 26 into the bloodstream. As the acoustic wave travels through the blood, the red cells in the blood back-scatter acoustic energy and generate a Doppler shifted signal which returns to the transducer 26. The returned acoustic energy is converted back into electrical energy by the transducer 26. This electrical signal from the transducer 26 is then amplified by the receiver 66 and is supplied to mixers 67 and 68.

The output $f_0$ from the oscillator is also supplied to a phase shifter 69 which generates two output signals which are sine and cosine waves that have a 90 degree phase shift between them. The cosine output from the phase shifter 69 is supplied to the mixer 67 whereas the sine output from the phase shifter 69 is supplied to the mixer 68. The use of two such signals which are 90 degrees shifted in phase provides a system which is quadrature based and makes it possible to distinguish between forward and reverse flow.

It should be appreciated that, if desired, other signal processing techniques well-known to those skilled in the art can be utilized for obtaining directional flow measurement as, for example, an offset frequency could be utilized. Alternatively separation of forward and reverse flows by single side band phasing techniques can also be employed. It should also be appreciated in those situations where the flow direction is known or for example is known to be unidirectional, simplified electronic circuitry can be utilized. It should also be appreciated that other signal processing techniques, such as spectral analysis, may be utilized to estimate the first movement of the Doppler signal.

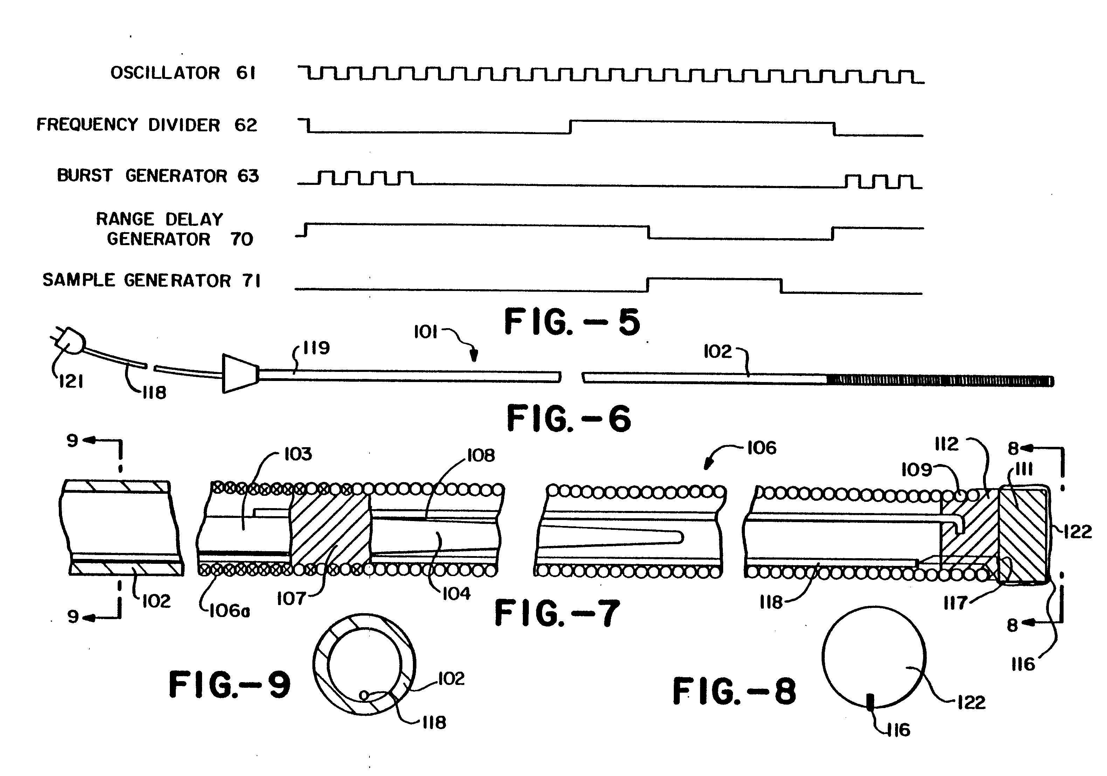

The output from the frequency divider 62 is also supplied to a range delay generator 70 which supplies its output to a sample generator 71. The output of the range delay generator 70 is in the form of a pulse which begins at the same time that the burst generator 63 begins the transducer drive burst and which ends at a time determined by the range input 70a to the range delay generator 70 as shown in Figure 5. The sample generator 71 generates a pulse which begins at the output end of the pulse range delay generator 70 and which has a pulse width equal to N3 cycles of frequency $f_0$. The relationship between the output signals from the oscillator 61, the frequency divider 62, the burst generator 63, the range delay generator 70 and the sample generator 71 are illustrated in the timing diagram shown in Figure 5. In general N2 and N3 are made equal for optimum signal-to-noise performance, although they are shown as separate values in Figures 4 and 5.

The mixers 67 and 68 generate output signals that are a product of their respective input signals. The output signals from the mixer 67 and 68 are supplied to sample gates 72 and 73. The sample gate 72 may be a "sample and hold" circuit which generates an output which follows the input from the mixer 68 during the pulse time from sample generator 71 and which holds the last value from the time the pulse ends until the next pulse. Alternatively the gate 72 can be a "gate, integrate and hold" circuit which generates an output proportional to the time integral of the output signal from the mixer 68 over the period of the sample pulse from sample generator 71. The principal difference between such two forms of the gate 12 relates to the

particular shape of the sample volume inside the blood vessel through which the volume flow is measured. The sample gate 73 is identical to the sample gate 72 and performs an equivalent operation on the output signal from the mixer 67.

The outputs from the sample gates 72 and 73 are fed into filters 74 and 75 respectively. The filters 74 and 75 are combinations of low pass and high pass filter sections and are utilized to determine the effective blood velocity ranges over which the volume flow measurements are to be made. The low frequency cutoff of the high pass filter section determines the lowest velocity included in the flow calculation and rejects the signals from slow moving vessel walls. The high frequency cutoff of the low pass filter is generally adjusted to be slightly less than one-half of the system repetition rate, as determined by the frequency divider 62. The filter 73 and 74 may also include compensation for the "(sine x)/(x)" effect of the sample gate 72 and the sample gate 73.

The outputs from the filters 74 and 75 are supplied to differentiators 76 and 77 which supply their outputs to multipliers 78 and 79 with the outputs of the multipliers 78 and 79 being supplied to a summation block 80. The differentiators 76 and 77, the multipliers 78 and 79 and the summation block 80 constitute a "first moment detector" which generates an output proportional to the spectral first moment of the two input signals from the filters 74 and 75. The differentiators 16 and 17 generate output signals proportional to the time derivative of their respective input signals. The multipliers 78 and 79 generate output signals proportional to the product of their respective input signals. The summation block 80 generates an output signal proportional to the difference between the output signal from the multiplier 78 and the output signal from the output signal 79. The first moment detector hereinbefore described has been known as a "cross-correlation detector". Other first moment detectors such as "square-root-of-frequency" versions can be utilized. The first moment detector which is shown in Figure 4 has been chosen because of its better signal-to-noise performance. Alternatively, the first moment may be calculated from a spectral analysis of the Doppler shift frequency.

The output of the summation block 80 supplies a first moment signal. The first moment signal which is supplied from the summation block 80 is applied to a normalization block 81 which receives calculated correction factor signal from a block 82. The correction factor which is supplied is hereinafter described.

The output from the normalization block 81 is supplied to a filter 83. The filter determines the final bandwidth of the volume flow output calculation. By way of example for bandwidths in the 1 to 50 hertz range, the flow output follows the instantaneous value of the volumetric blood flow. For lower bandwidths in the 0 to 1 hertz range, the flow output is proportional to the average (mean) volume of blood flow. The volume flow output signal from the filter 83 is supplied to the digital display 56 to provide an output digital display in the form of liters per minute. This same volume flow output signal can be supplied to the oscilloscope 58 and the strip chart 59 if desired.

The mathematical calculations which are performed by the circuitry shown in Figure 4 are based on the following. The instantaneous volumetric flow or volume flow $\overset{\circ}{Q}$ (t) of a fluid across a surface, S, is defined as:

$$\overset{\circ}{Q}(t) = \int_S v(t) \bullet dA \qquad (1)$$

where $\vec{v}$ (t) is the velocity vector of the fluid at any point on surface S, $\vec{d}A$ is the elemental area vector perpendicular to the surface and the symbol "$\bullet$" indicates the vector dot-product. The spectral first moment, MI, of the received power is defined as

$$MI = \int f S(f) df \qquad (2)$$

where f is frequency and S(f) is the power spectrum of the received Doppler-shifted signal.

The above calculations have been based upon the assumption of a uniform acoustic power density in the ultrasonic beam. It is also assumed that a first-order, independent scattering process takes place, that the acoustic wavelength is much greater than the scattering particle size and that there is a uniform distribution of scatterers throughout the acoustic beam. Using these assumptions the spectral first moment of the power spectrum of the received signal is:

$$MI = I(R) \, \eta \, T(R) \, \overset{\circ}{Q} \qquad (3)$$

where I(R) is the transducer sensitivity at range R for the ultrasonic beam, $\eta$ is the volumetric scattering coefficient of blood and T(R) is the round trip transmission efficiency representing the effects of attenuation of the acoustic power caused by the blood.

Equation (3) shows that the volume flow of blood can be estimated by first measuring the spectral first moment of the power spectrum of the received Doppler-shifted signal and then correcting the measurement in accordance with the three parameters, I(R), $\eta$ and T(R). Different methods are available for the determination of these parameters. One method is to separately determine the parameters. The first factor, I(R) can be measured in conventional beam pattern test facilities. The second factor, $\eta$, can be measured experimentally and is not expected to deviate from the experiments due to the consistency of the hematocrit level. The third factor, I(R), can also be measured experimentally. Of the three parameters, I(R) and $\eta$ would

normally remain constant for a given value of R.

In most flow measurement situations, the value for T(R) would not be well controlled and would introduce significant errors. It is for this reason that a single intravascular probe is utilized in conjunction with a well-controlled intervening medium such as blood between the transducer and the sample volume. Furthermore, since the attenuation in the blood is significantly less than the attenuation in tissue, the effect of the T(R) term is significantly less than for cutaneous flowmeters. In addition since the attenuation in the blood is less than in tissue, the sensitivity of the final estimate of blood flow to the actual coefficient of attenuation is significantly less. Additionally, since the attenuation in blood is significantly less than attenuation in tissue, any signals from other vessels outside the vessel of interest will be so strongly attenuated as to become insignificant.

Another method for determining the three above-identified parameters is to combine all three in a single calibration. This can be accomplished by comparing Doppler determined flow with the actual flow output, measured for example by collecting the flow volume in a measuring device, and thus determining the calibration relationship between MI and Q.

From the foregoing it can be seen that relatively accurate measurement of volumetric blood flow can be obtained in a vessel without the use of a second transducer or a second ultrasonic beam. Additionally, accurate volumetric flow measurements can be made which utilize a single transducer which are independent of the angle and position of the transducer in the vessel. By selecting the appropriate frequencies and the appropriate size for the transducer it is possible to obtain a beam divergence angle of greater than 10°, as for example, a range from 15° to 120° in order to ensure that at a selected range depth in the far field region a relatively uniform beam will be produced over the entire cross-sectional area of the vessel. By utilizing previously calculated scale factors in the correction factor block 82, the first moment of flow can be utilized to calculate instantaneous volumetric flow through the vessel before the balloon dilation procedure. As soon as this measurement has been made, the balloon can be advanced into the stenosis and inflated to enlarge the stenosis. After this has been accomplished, the balloon 21 can be withdrawn proximal to the stenosis. Another flow measurement can thereafter be made to ascertain the volumetric blood flow after the dilation procedure. If the volumetric blood flow is inadequate, the balloon can be again advanced into the stenosis and another dilation can be performed. Alternatively, if a larger balloon size is desired, an exchange wire can be substituted for the guide wire 46 and a dilatation catheter having a larger diameter balloon can be utilized to cause a greater enlargement of the stenosis in the vessel. Thereafter, another volumetric flow measurement can be made.

Rather than utilizing a balloon dilatation catheter for the flexible elongate transducer carrying device, a guide wire 101 of the type shown in Figures 6, 7, 8 and 9 can be utilized. Such a guide wire is comprised of a flexible elongate element 102 which can be in the form of a hypo tube 102 having a suitable outside diameter as, for example, .016 inches, and having suitable wall thickness as, for example, .002 inches. In order to provide additional rigidity and torqueability for the guide wire 101, a core wire 103 formed of a suitable material such as stainless steel is provided. The core wire 103 can have a suitable diameter as, for example, .008 inches and extends through the hypo tube 102. Its distal extremity 104 is tapered for a distance of approximately 15 centimeters from a diameter of .008 inches to a diameter of .003 inches. The distal extremity 104 extends beyond the hypo tube 106 and extends into a coil spring 106 which is secured to the hypo tube 102 in an appropriate manner such as by brazing. The coil spring 106 is formed of two parts, a part 106a which is formed of stainless steel and the other part 106b of a more opaque material such as a platinum tungsten alloy or other material as described in United States Letters Patent No. 4,538,622. At the region where the two portions 106a and 106b are screwed together, the spring is bonded to the core wire 103 by solder or an epoxy 107. A safety wire or shaping ribbon 108 is provided. It is formed of a suitable material such as stainless steel ribbon and has a cross-sectional dimensions of .001 inches x .003 inches. The safety ribbon or shaping ribbon 108 extends from the solder or epoxy joint 107 to the distal extremity 109 of the coil spring 106. A transducer 111 of a suitable type as, for example, a piezoelectric crystal of the type hereinbefore described is carried by the distal extremity 109 of the coil spring 106 and is secured thereto by suitable means such as a tungsten-oxide loaded epoxy 112. As can be seen, the shaping wire 108 extends into the epoxy 112. Front and rear contacts 116 and 117 are provided on the transducer 111 and are connected to a two conductor wire 118 which extends rearwardly and interiorly of the spring 106 and extends into the hypo tube 102 between the core wire 103 and the interior of the hypo tube 102. The wire 118 extends out the distal extremity 119 of the hypo tube 102 and is connected to a male connector 121. The distal extremity of the hypo tube 119 can be secured to the core wire by suitable means such as an epoxy. The surface of the crystal serving as a transducer 11 can be coated with a suitable protective material such as a urethane

coating 122. As shown, the spring 106 can extend for a predetermined distance, as for example, 1.5 centimeters beyond the tapered distal extremity 104. The portion 106b of the coil 106 can have a suitable length as, for example, 3 centimeters.

The guide wire 101 can have a suitable overall length, as for example, 175 centimeters. The crystal transducer 111 can have a suitable diameter as, for example .019 inches.

By providing a guide wire of this size, it is possible to utilize a guide wire in connection with conventional balloon dilatation catheters to perform angioplasty procedures.

Use and operation of the guide wire 101 in conjunction with the system and apparatus hereinbefore described is very similar to that described in conjunction with the use of the balloon dilatation catheter. After the guide wire and the balloon dilatation catheter have been advanced into a region adjacent to the stenosis, a volumetric flow measurement can be made by attaching the male connector 121 to the female connector 51 of the flow meter 13. A reading will be given on the digital display 56 giving an indication of the volumetric blood flow. After a dilatation procedure has been accomplished, the balloon dilatation catheter can be withdrawn proximal to the stenosis and a volumetric flow measurement can again be made by withdrawal of the guide wire to a position proximal to the stenosis and in approximately the same position it was in during flow measurement prior to dilation of the stenosis.

Typically for use with the guide wire carried transducer, the transducer would have a suitable frequency as, for example, 10 MHz and a diameter of 0.5 millimeters to produce a beam divergence of approximately 20° which will again produce a far field uniform beam capable of isonifying a 2.5 millimeter vessel at a range gate depth of 10 millimeters. Thus again, it can be seen that this makes possible instantaneous volumetric flow measurements before and after an angioplasty procedure.

It is apparent from the foregoing that there has been provided a system, apparatus and method for measuring volumetric flow of blood in a vessel. This can be accomplished by the use of a single transducer positioned intravascularly to produce a beam of uniform insonification which encompasses the entirety of the blood vessel. By utilizing a calculated correctional factor applied to the signal obtained from a first moment detector, accurate measurement of volumetric blood flow may be obtained. These blood flow readings are obtained with little or no signal interference from vessels beyond the boundaries of the wall of the vessel in which the transducer is located. The extremely small transducers which are utilized make it possible to produce a far field uniform beam which encompasses the entirety of the vessel lumen. Volumetric flow measurements obtained using this uniform beam are substantially independent of the angle and orientation of the transducer in the vessel and of the flow profile within the blood vessel.

It should be appreciated that although the present invention has been described particularly for use in the measuring of the flow of blood in a vessel, the present invention also can be utilized for measuring other liquids in other types of conduits if desired.

## Claims

1. In a system for measuring the volumetric flow of blood in a vessel of a patient, a transducer adapted to be positioned in a vessel in a patient, means for supplying electrical energy to the transducer, the transducer producing a substantially uniform ultrasonic beam which encompasses the vessel, the transducer receiving ultrasonic energy back scattered from the red blood cells, a first moment detector and means supplying a signal from the transducer to the first moment detector and applying a calculated correctional factor to the output of the first moment detector to provide an electrical output representing volume flow.

2. A system as in Claim 1 wherein the transducer operates in the range 1 to 20 MHz and has a diameter ranging from .10 millimeters to 5.0 millimeters.

3. A system as in Claim 2 wherein the range depth is on the order of 1 to 40 millimeters.

4. A system as in Claim 2 wherein the beam divergence ranges from 5 to 120°.

5. A system as in Claim 1, wherein the first moment is calculated from a spectral analysis of the Doppler shift frequency.

6. In a method for measuring volumetric flow of a liquid in a vessel by the use of a single transducer positioned within the vessel, supplying an electrical signal to the transducer to cause it to provide an ultrasonic beam pattern which is substantially uniform across the cross-sectional area of the vessel, receiving back scattered ultrasonic energy having a Doppler shift frequency with the transducer and converting it to an electrical signal and using the electrical signal to calculate a first moment and applying a calculated correctional factor to the the first moment to provide an output which indicates the volume of flow of liquid in the vessel.

7. A method as in Claim 6 wherein the vessel has a diameter of between 1 millimeter and 35 millimeters.

8. A method as in Claim 6 wherein the beam divergence ranges from 5° to 120°.

9. A method as in Claim 6 wherein the range depth is on the order of 1 to 40 millimeters.

10. A method as in Claim 6 wherein the first moment is calculated from a spectral analysis of the Doppler shift frequency.

11. A method as in Claim 6 wherein a volumetric flow measurement is made before and after balloon dilatation.

12. In a device for use in measuring volumetric flow of blood in a vessel, a flexible elongate element having proximal and distal extremities; a coil spring carried by the distal extremity of the flexible elongate element, the coil spring having a distal extremity, a crystal carried by the distal extremity of the coil spring and conductive means connected to the crystal and extending interiorly of the coil spring and of the flexible elongate element.

13. A device as in Claim 12 wherein said flexible elongate element is in the form of a hypo tube and wherein the conductive means extends through the hypo tube.

14. A device as in Claim 12 wherein said flexible elongate element is in the form of a guide wire.

0 286 359

11

13

FLOW METER
DIGITAL DISPLAY

56

LITERS/MIN.

53

51    48

52    43    34    36    37    12

46    21    46

46

42    44    41    38    38

# FIG.-1

57

58    59

32    21

28    31

26

# FIG.-3

22    34    21    27    31    33    3

19    3

32

28

26

18    16    38    49    38    17    16    29    3

16

# FIG.-2

FIG. —4

0 286 359

OSCILLATOR 61

FREQUENCY DIVIDER 62

BURST GENERATOR 63

RANGE DELAY GENERATOR 70

SAMPLE GENERATOR 71

FIG.—5

FIG.—6

FIG.—7

FIG.—8

FIG.—9